# EUROPEAN PATENT APPLICATION

(11) **EP 4 113 534 A1**
(43) Date of publication of application: **04.01.2023**
(21) Application number: 22181280.3
(22) Date of filing: 27.06.2022
(51) Int. Cl.: G16H 40/40, G16H 40/67, H04W 12/06, A61G 7/00

(54) **REMOTE DIAGNOSTICS FOR PATIENT BEDS**

(30) Priority: 28.06.2021 US 202163215612 P
(71) Applicant: Hill-Rom Services, Inc., Batesville, IN 47006-9167 (US)
(72) Inventor: BENZ, Eric D., Batesville, 47006-9167 (US); COMPARONE, Nicholas, Batesville, 47006-9167 (US); CRAWFORD, Barbara, Batesville, 47006-9167 (US); McCAIG, Steven V., Batesville, 47006-9167 (US); OJHA, Unnati, Batesville, 47006-9167 (US); ROBACIU, Tudor, Batesville, 47006-9167 (US); TADIPATRI, Vijay Aditya, Batesville, 47006-9167 (US)
(74) Representative: Findlay, Alice Rosemary

(57) **Abstract**

A system for remote diagnostics of medical devices in a healthcare network includes at least one medical device having an associated serial number. A service platform is in communication with the at least one medical device. A manufacturer database is in communication with the service platform. A remote service system is in communication with the service platform. The at least one medical device stores unique device credentials. The remote service system identifies the at least one medical device based on the unique device credentials.

## Description

This application claims priority to U.S. Provisional Application No. 63/215,612, filed June 28, 2021, which is expressly incorporated by reference herein.

The present disclosure relates to medical devices and, more particularly, to a system for remote diagnostics for medical devices.

When some wireless medical devices are installed in a hospital or other healthcare facility, the devices are configured with credentials so that the device can connect to remote services. The configuration step requires service technicians to go from device-to-device and set up the credentials and other configuration parameters manually. This process can add up to hours of work for service technicians. Furthermore, once a device is connected to a remote cloud server, it is important to know which facility/customer the device belongs to so that service technicians can assign/notify the proper audience about the status of the device. This process currently requires remote service technicians to refer to a different server, locate the serial number of the bed on that server and glean the ownership/customer's information from that server. This manual process can be time consuming and can also introduce human errors.

Additionally, service personnel are tasked with performing service tasks such as making repairs, performing calibrations, and preventative maintenance to many different devices. Each device that needs servicing will have separate and unique service procedures to accomplish the service tasks. These service procedures can exist in paper or various electronic formats and are provided to service personnel to store and maintain for reference purposes should these procedures be needed in the future. The passage of time and need to organize and store these procedures can make it difficult to retrieve the proper procedure at the appropriate time when the service is needed on the device. This leads to service inefficiency and wasted cost for companies providing service such as device manufacturers, hospitals, and related maintenance service providers.

When a device issue occurs, there is rarely an accurate picture of the activity that preceded the failure. Often, only the diagnostic code on the device is available to indicate to the service technician what the problem is. The diagnostic code does not help determine why the failure occurred in the first place. Even when the failure is observed in real time, the only avenue for determining preceding information is to talk with individuals who were working with the device. These discussions can yield inconsistent information and/or leave out key details. It is not practical to expect someone to recollect every activity that occurred on the device and the sequence in which the activities occurred.

Retracing the history of activity on a device is critical to performing a robust root cause analysis investigation. Simply making a repair by replacing a component after receiving a diagnostic code could potentially mask an underlying problem. Even when a design issue is suspected, it can be very challenging to try and recreate the issue. Piecing together partial information and trying to find the exact sequence of events that precipitated the issue is required. Being able to reproduce any failure is critical to identifying the root cause.

The present disclosure includes one or more of the following features alone or in any combination.

According to a first aspect of the disclosed embodiments, a system for remote diagnostics of medical devices in a healthcare network includes at least one medical device having an associated serial number. A service platform is in communication with the at least one medical device. A manufacturer database is in communication with the service platform. The manufacturer database includes data related to the at least one medical device. A remote service system is in communication with the service platform. The at least one medical device sends a registration request to the manufacturer database via the service platform. The registration request includes the serial number of the at least one medical device. Upon recognizing the serial number of the at least one medical device, the manufacturer database returns unique device credentials to the at least one medical device. The at least one medical device stores the unique device credentials. The remote service system identifies the at least one medical device based on the unique device credentials.

In some embodiments of the first aspect, the at least one medical device may send a create device request to the remote service system via the service platform. The remote service system may create a local identification for the at least one medical device. The at least one medical device may store the local identification. The at least one medical device may send an error code to the remote service system via the service platform. The remote service system may identify the at least one medical device based on the unique device credentials. The remote service system may open a service order based on the error code and the unique device credentials. The remote service system may identify parts necessary to repair the at least one medical device based on the error code and the unique device credentials. The remote service system may notify a technician of the service order.

Optionally, in the first aspect, the at least one medical device may request a software update from the remote service system via the service platform. The remote service system may push the software update to the at least one medical device. The remote service system may push the software update to a plurality of medical devices that includes the at least one medical device. The software update may be applied to the at least one medical device by a technician at the at least one medical device.

It may be desired, in the first aspect, that the remote service system periodically determines whether the at least one at medical device requires a software update. The remote service system may push the software update to the at least one medical device via the service platform. The at least one medical device may store data related to device events. The device events may include at least one of user alarms, change of bed position, user input actions, connectivity events, bed errors, change in patient position and weight, run time on components, surface state, nurse call status, battery power status, and battery run time. The data related to device events may be transmitted to the remote service system via the service platform after a predetermined period of time. The data related to device events may be transmitted to the remote service system via the service platform after a predetermined number of device events. The data related to device events may be transmitted to the remote service system via the service platform when an error code is transmitted to the remote service system. The data related to device events may be stored until the error code is cleared. The data related to device events may include data related to device events that occurred within a range of 1 hour to 2 hours prior to the error code. The data related to device events may include data related to approximately 50 -100 device events prior to the error code.

According to a second aspect of the disclosed embodiments, a method of remotely servicing medical devices in a healthcare network includes sending a registration request from at least one medical device to the manufacturer database via a service platform. The registration request includes the serial number of the at least one medical device. Upon recognizing the serial number of the at least one medical device, unique device credentials are returned from the manufacturer database to the at least one medical device. The method also includes storing the unique device credentials at the at least one medical device. The method also includes identifying the at least one medical device based on the unique device credentials at a remote service system. The method also includes sending a create device request from the at least one medical device to the healthcare network via the service platform. The method also includes creating a local identification for the at least one medical device at the healthcare network. The method also includes storing the local identification at the at least one medical device.

In some embodiments of the second aspect, the method also include sending an error code from the at least one medical device to the remote service system via the service platform. The method may also include identifying the at least one medical device at the remote service system based on the unique device credentials. The method may also include opening a service order at the remote service system based on the error code and the unique device credentials. The method may also include identifying at the remote service system parts necessary to repair the at least one medical device based on the error code and the unique device credentials. The method may also include requesting a software update from the remote service system via the service platform. The method may also include pushing the software update from the remote service system to the at least one medical device. The method may also include pushing the software update to a plurality of medical devices that includes the at least one medical device.

Optionally, in the second aspect, the method may also include periodically determining at the remote service system whether the at least one at medical device requires a software update. The method may also include pushing the software update to the at least one medical device via the service platform. The method may also include storing data related to device events at the at least one medical device. The method may also include transmitting the data related to device events to the remote service system via the service platform at least one of after a predetermined period of time, after a predetermined number of device events, and when an error code is transmitted to the remote service system. The data related to device events may include data related to device events that occurred within a range of 1 hour to 2 hours prior to the error code. The data related to device events may include data related to approximately 50 -100 device events prior to the error code. The method may also include storing the data related to device events until the error code is cleared.

According to a third aspect of the disclosed embodiments, a system for remote diagnostics of medical devices in a healthcare network includes at least one medical device having an associated serial number. A service platform is in communication with the at least one medical device. A manufacturer database is in communication with the service platform, the manufacturer database including data related to the at least one medical device. A remote service system is in communication with the service platform. The at least one medical device receives unique device credentials from the manufacturer database. The at least one medical device receives a local identification for the at least one medical device from the remote service system. The at least one medical device send an error code to the remote service system via the service platform. The remote service system identifies the at least one medical device based on the unique device credentials.

In some embodiments of the third aspect, the remote service system may identify parts necessary to repair the at least one medical device based on the error code and the unique device credentials. The at least one medical device may request a software update from the remote service system via the service platform. The remote service system may push the software update to the at least one medical device. The remote service system may push the software update to a plurality of medical devices that includes the at least one medical device. The remote service system may periodically determine whether the at least one at medical device requires a software update. The remote service system may push the software update to the at least one medical device via the service platform.

Optionally, in the third aspect, the at least one medical device may store data related to device events. The device events may include at least one of user alarms, change of bed position, user input actions, connectivity events, bed errors, change in patient position and weight, run time on components, surface state, nurse call status, battery power status, and battery run time. The data related to device events may be transmitted to the remote service system via the service platform at least one of after a predetermined period of time, after a predetermined number of device events, and when an error code is transmitted to the remote service system. The data related to device events may include data related to device events that occurred within a range of 1 hour to 2 hours prior to the error code. The data related to device events may include data related to approximately 50 -100 device events prior to the error code. The data related to device events may be stored until the error code is cleared.

Additional features, which alone or in combination with any other feature(s), such as those listed above and/or those listed in the claims, can comprise patentable subject matter and will become apparent to those skilled in the art upon consideration of the following detailed description of various embodiments exemplifying the best mode of carrying out the embodiments as presently perceived.

The disclosure will now be further described by way of example with reference to the accompanying drawings, in which:
Fig. 1 is a schematic view of a network that includes a patient support apparatus, wherein the network is configured for remote diagnostics of the patient support apparatus;
Fig. 2 is a schematic view of a network that includes medical devices, wherein the network is configured for remote diagnostics of the medical devices;
Fig. 3 is a schematic illustration of a method for registering a medical device using the network of Fig. 1 or Fig. 2;
Fig. 4 is a schematic view illustrating a continuation of the method shown in Fig. 3;
Fig. 5 is a schematic view of a method for determining ownership of a medical device using the network of Fig. 1 or Fig. 2;
Fig. 6 is a schematic illustration of a method for requesting a firmware update for a medical device using the network of Fig. 1 or Fig. 2;
Fig. 7 is a schematic view of a medical device of the network of Fig. 1 or Fig. 2 and information that the medical device shares with the network; and
Fig. 8 is a schematic illustration of a method for troubleshooting a medical device using the network of Fig. 1 or Fig. 2.

While the concepts of the present disclosure are susceptible to various modifications and alternative forms, specific exemplary embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the scope of the invention as defined by the appended claims.

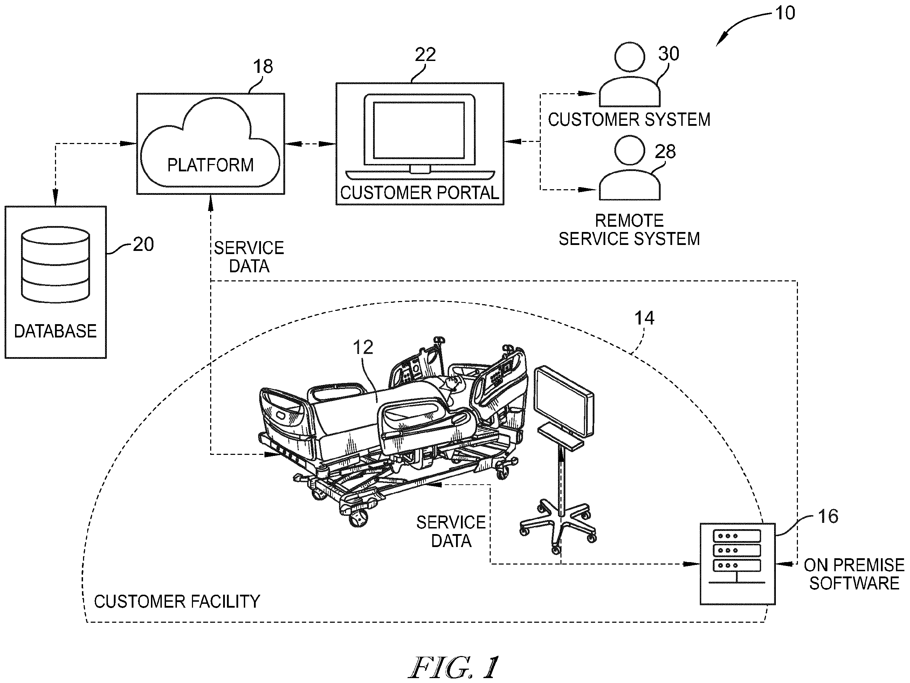

Referring to Fig. 1, a network 10 is configured for remote diagnostics of a medical device 12. In the exemplary embodiment, the medical device 12 is a patient support apparatus illustrated as a bed. In some embodiments, the medical device 12 may be any medical device in a healthcare facility. The device 12 is located at a facility 14, for example a healthcare facility. It should be readily appreciated that the facility 14 may include multiple Local Area Networks ("LANs") and/or Wide Area Networks ("WANs") that are operably coupled to one another via routers, switches, hubs, gateways, proxies, and/or firewalls (not shown). The device 12 has been purchased from a manufacturer or supplier for use in the facility 14. The network 10 is configured to enable remote diagnostics of the device 12 at the manufacturer or supplier facility. For example, the network 10 recognizes the device and stores unique data, e.g. a serial number, model number, etc. The unique data is associated with the device 12 and the facility 14 to enable remote diagnostics of the device 12, as described in more detail below. On-premise, e.g. at the facility 14, software 16 is stored on the device 12 to enable communication with the network 10.

The network 10 includes a service platform 18 that is located remotely from the healthcare facility 14. The facility 14 is communicatively coupled to the service platform 18 via the cloud or Internet. The device 12 is in wireless communication with the service platform 18. A manufacturer database 20 is also in wireless communication with the platform. The manufacturer database 20 stores data related to a plurality of medical devices, including the medical device 12. The data includes medical device serial numbers, medical device model numbers, and ownership data related to each device, for example the name and address of the healthcare facility that purchased the device.

A customer portal 22, operated by a customer at a customer system 30 of the facility 14, is in wireless communication with the platform 18. The customer portal 22 may include a remote computer located at the facility 14. In some embodiments, the customer portal 22 is located off-site at another facility owned by the facility 14. The customer portal 22 enables the customer to access information regarding the device 12, request maintenance on the device 12, request parts for the device 12, etc. The customer system 30 enables the customer to view their own device service data. Remote update configurations and remote upgrade firmware may also be accessed by the customer at the customer system 30. In some embodiments, the customer may access pre-defined reports and logs related to the device 12 at the customer system 30.

A remote service system 28 of the manufacturer/supplier also has access to the customer portal 22. Customer service may accesses the customer portal 22 to respond to requests from the customer through the remote service system 28. In some embodiments, the customer service may remotely attend to maintenance on the device 12 over the network 10 by accessing data on the remote service system 28. At the remote service system 28, customer service may view all device service data and review notifications related to the device 12. Customer service may also deploy updates to the device 12 through the remote service system 28. At the remote service system 28, customer service may also generate reports and obtain logs related to the device 12.

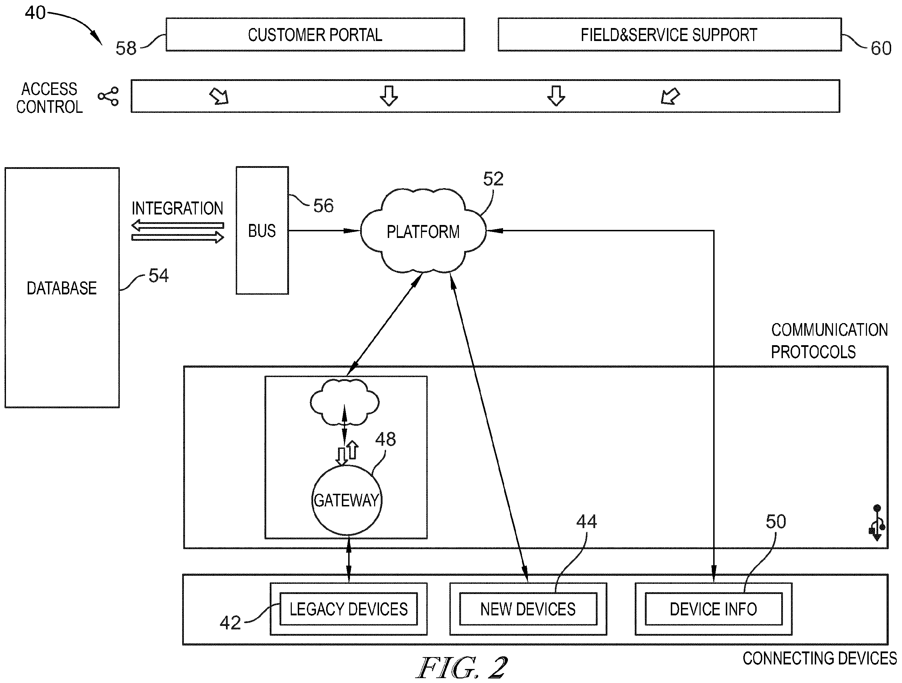

Referring to Fig. 2, another example of a network 40 for remote diagnostics includes legacy medical devices 42 that are not equipped with wireless technology and wireless medical devices 44 located in the facility 14. It should be readily appreciated that the facility 14 may include multiple Local Area Networks ("LANs") and/or Wide Area Networks ("WANs") that are operably coupled to one another via routers, switches, hubs, gateways, proxies, and/or firewalls (not shown). The devices 42 and 44 may be any device operable in the facility 14, e.g. beds, pulse oximeters, electrocardiograms, etc. The legacy medical devices 42 are electrically coupled to a wireless gateway 48 that enables wireless communication with the network 40. In some embodiments, the gateway 48 is operated through message queuing telemetry transport or application programming interface protocols. As such, the users at the facility 14 may operate the devices 42 over the network 40 without the need to replace devices 42 that are still operable. A wireless gateway 50 accesses the data related to the devices 42 and 44. In some embodiments, the gateway 50 is operated through message queuing telemetry transport or application programming interface protocols.

The devices 42, 44 and the gateway 50 are in communication with a service platform 52 that is remote from the facility 14. For example, the platform 52 may be located in the cloud, similar to platform 18. A manufacturer database 54 communicates with the platform 52 over a bus 56. In some embodiments, the bus 56 leverages existing middleware and/or application programming interface connectors. The manufacturer database 54 is similar to the database 20 and stores information related to the devices 42, 44.

A customer portal 58, similar to portal 22 communicates with the platform 52 to enable customer support, as described above. Additionally, a field service portal 60, similar to the remote service system 28, also communicates with the platform 52. The portal 60 may include a remote device located at a customer support center and/or a device carried by a field technician during visits to the facility 14 to perform maintenance on the devices 42, 44.

It should be noted that networks 10 and 40 utilize similar components for remote diagnostics. It will be appreciated that any of the components operable in network 10 may also be utilized in network 40. Likewise, any components operable in network 40 may also be utilized in network 10. Network 10 and network 40 are two exemplary embodiments of networks that may be used for remote diagnostics, as described below. Additional components for network 10 and network 40 may be contemplated.

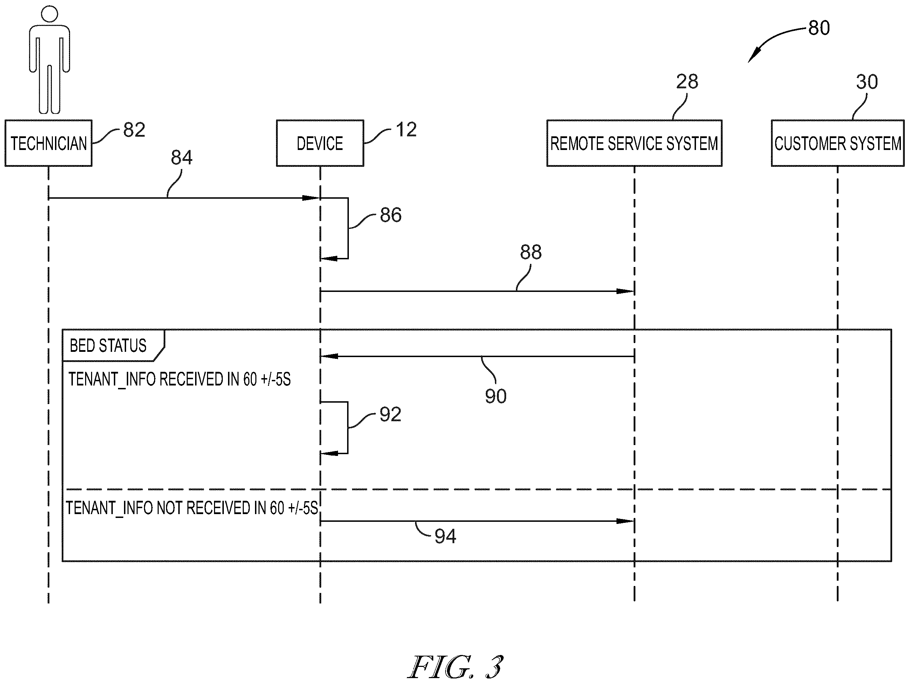

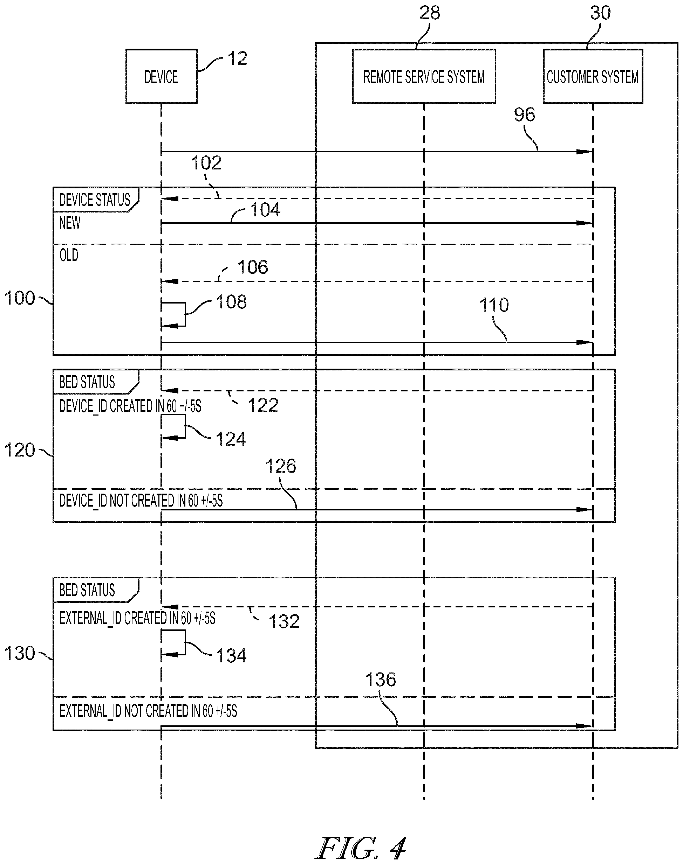

Referring now to Figs. 3 and 4, a method 80 for registering the device 12 is illustrated. Although the method 80 is discussed with relation to the network 10 shown in Fig. 1, it will be appreciated that the method 80 is equally applicable to network 40, shown in Fig. 2. It should also be noted that Fig. 4 is a continuation of Fig. 3. That is, once the steps of Fig. 3 and completed, the method 80 continues to the steps shown in Fig. 4. The method 80 takes place at the time that the device 12 is first installed in the facility 14. With regard to the legacy medical devices 42, shown in Fig. 2, the method 80 may be implemented retroactively.

Referring to Fig. 3, a technician 82 is present in the facility 14 when the device 12 is installed. As indicated by action 84, the technician 82 turns the device 12 on for the first time. The device 12 then wirelessly connects to the network 10, as indicated by action 86. Once a connection to the network 10 is achieved, the device 12 sends a registration request to the remote service system 28 over the platform 18, as indicated by action 88. The registration request includes device data that is unique to the device 12, e.g. a unique serial number associated with the device 12. The information may also include a model number.

After the remote service system 28 receives the request, one of two events occurs. In a first scenario, the device data matches the data stored in the database 20 and the initial request is returned, at action 90, including new device-specific credentials that identify the device 12. At action 92, the device-specific credentials are stored on the device 12. In a second scenario, the initial request is not recognized and, at action 94, the device 12 sends a second request over the network 10. The device 12 continues to send requests until the device 12 is recognized by the remote service system 28.

Once the device 12 is recognized by the remote service system 28, the method 80 continues to Fig. 4. After registering the device 12, the device 12 sends a request, as indicated by action 96, to request a unique Device ID from a customer system 30 of the facility 14. The Device ID enables the device 12 to be tracked and maintained within the facility 14. For a device 12 that is newly registered, the box 100 illustrates the steps taken to receive the Device ID. First, at action 102, the customer system 30 responds that the Device ID has not been found. The device 12 then sends a "Create Device ID" request to the customer system 30, at action 104. The customer system 30 returns a unique Device ID at action 106 and the device 12 stores the unique Device ID, at action 108. The facility records are then updated, at action 110, at the customer system 30.

Referring to box 120, if the Device ID is created within a predetermined period of time, the Device ID is returned to the device 12, at action 122, and, at action 124, the Device ID is stored in the device 12. In some embodiments, the predetermined period of time may be 55 seconds to 65 seconds. If the Device ID is not returned within the predetermined period of time, the device 12 resends the "Create Device ID" request at action 126. Once the Device ID is stored, a "Create External ID" request is sent from the device 12 to the customer system 30. The unique External ID includes facility data utilized to access the device 12 over the network 10. For example, the facility data may include a local identification related to a name of the facility 14, an address of the facility 14, and/or information related to the location of the device 12 in the facility 14.

Referring to box 130, if the External ID is created within a predetermined period of time, the External ID is returned to the device 12, at action 132, and, at action 134, the device 12 stores the External ID. In some embodiments, the predetermined period of time may be 55 seconds to 65 seconds. If the External ID is not created within the predetermined period of time, the "Create External ID" request is resent from the device 12 to the customer system 30, at action 136. After method 80 is completed, the device 12 has unique data stored thereon including the Device ID and the External ID. Accordingly, this data may be shared over the network 10 and shared with the technician 82 to assist in maintaining the device 12, both in person and remotely over the network 10.

Accordingly, before a device 12 is installed in the facility 14, the device 12 is loaded with default bootstrap credentials and default Wi-Fi network parameters. These parameters may be installed on the device 12 at the manufacturer or supplier. During the installation process in the facility 14, the technician 82 sets up an installation network using the stored Wi-Fi network information. When the device 12 powers-up, the device 12 connects to the internet and a registration specific service in the remote service system 28 using the default credentials. During the connection to the remote service system 28, the device 12 sends its serial number to the remote service system 28. The remote service system 28 registers the device 12 using its serial number and provides the device 12 with the Device ID to use on all future connections. The customer system 30 also connects to the database 20 and uses the serial number of the device 12 to request the External ID related to the device 12. The External ID is saved in the customer system 30 and also pushed down to the device 12. This completes the provisioning process.

The network 10 uses default credentials to communicate to the remote service system 28 and retrieve device-specific credentials for all future communication/connections with the platform 18. Upon receiving the device-specific credentials, the device-specific credentials are stored in device 12. In some embodiments, the device-specific credentials are stored as plain text files. In some embodiments, the device-specific credentials are encrypted. Accordingly, the device 12 is registered in a one-time event and no additional configuration is necessary for the device 12 during installation in the facility 14.

Moreover, the customer system 30 creates device representation through the Device ID. The Device ID operates a as primary identifier on the customer system 30. The Device ID is also stored on the device 14. The External ID, which maps to the Device ID, is created by the device 14 for easily referring to the device 14 at the customer system 30. The creation of the Device ID and the External ID is a one-time event. As such, all data coming in the device 12 is associated with the Device ID and External ID.

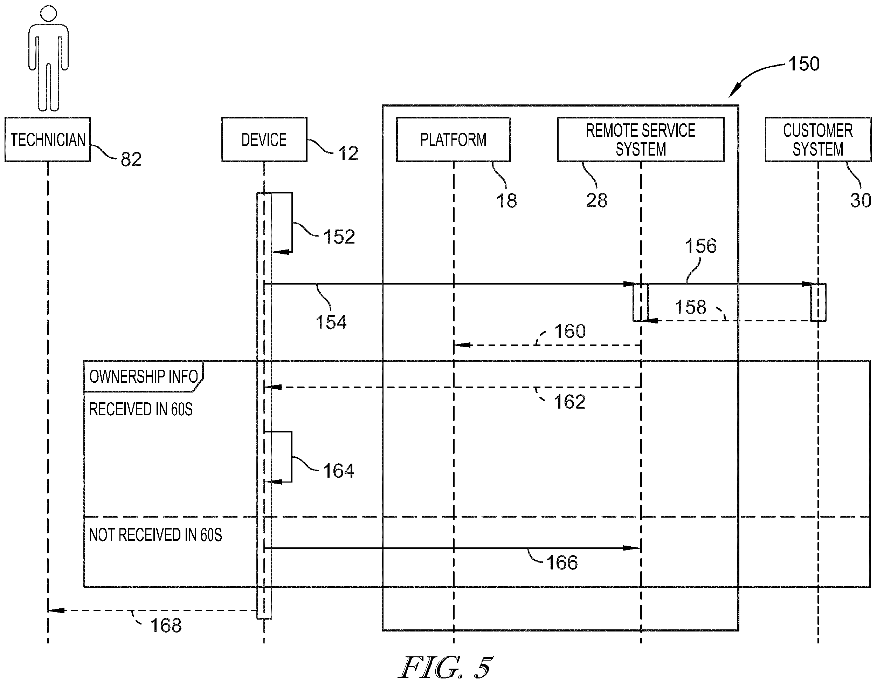

Fig. 5 illustrates a method 150 that enables the technician 82 to acquire the unique data related to the device 12 to assist in maintenance of the device 12. Although the method 150 is discussed with relation to the network 10 shown in Fig. 1, it will be appreciated that the method 150 is equally applicable to network 40, shown in Fig. 2. The method 150 is implemented when a service request for the device 12 has been issued or when the device 12 is initially installed. The method 150 is implemented after the device 12 has been successfully registered, at action 152. That is, the method 150 is implemented after the Device ID and External ID have been associated with the device 12. At action 154, the Device ID and External ID are requested by the technician 82. The remote service system 28 communicates with the customer system 30 and requests the Device ID and External ID, at action 156. The Device ID and External ID is returned to the remote service system 28, at action 158, and Device ID and External ID are pushed to the platform 18, at action 160 to record and generate a service order. If the database 20 sends the Device ID and External ID back to the device 12, at action 162, within 60 seconds, the Device ID and External ID are stored on the device 12, at action 164, and accessible by the technician 82 at the device 12. If the Device ID and External ID are not sent back to the device 12 within a predetermined period of time, the device 12 sends additional requests for Device ID and External ID, at action 166. In some embodiments, the predetermined period of time is 60 seconds. After the Device ID and External ID are stored in the device 12, the Device ID and External ID are displayed to the technician 82, at action 168.

The method 150 enables the technician 82 to determine which customer, e.g. facility 14, owns the device 12. The technician 82 may use the serial number of the device 12 to determine ownership from the database 20 and/or the customer system 30 through the Device ID and External ID. Determining ownership of the device 12 facilitates locating the device 12 and/or categorizing devices 12 according to customer. In some embodiments, devices 12, when booting up, request the Device ID and External ID in case the Device ID and External ID have changed.

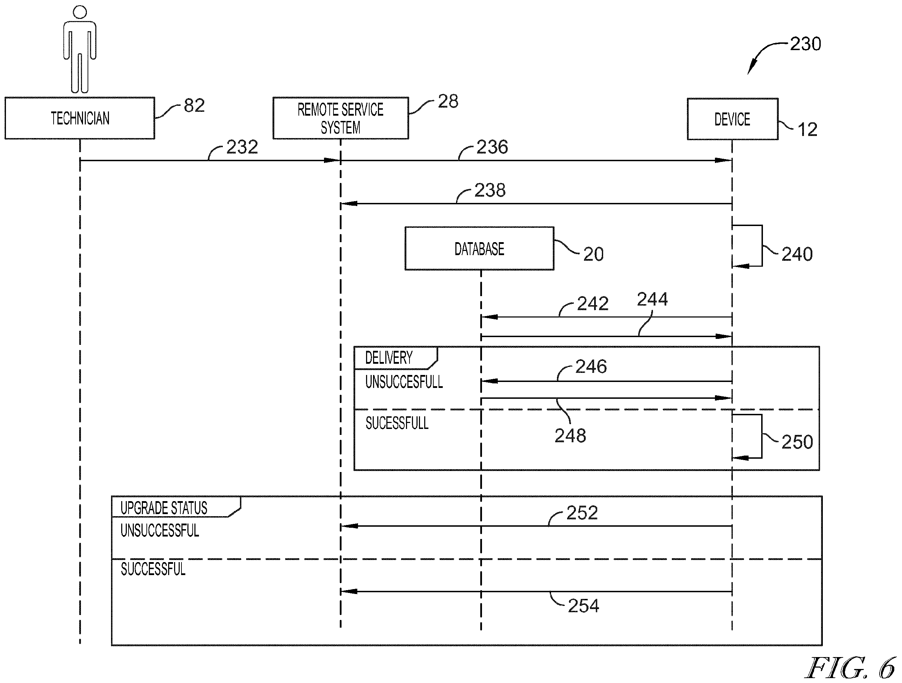

Referring now to Fig. 6, a method 230 for remotely updating the firmware of the device 12 starts when a remote technician 82 selects a file and requests the firmware upgrade at action 232. Although the method 230 is discussed with relation to the network 10 shown in Fig. 1, it will be appreciated that the method 230 is equally applicable to network 40, shown in Fig. 2. The firmware upgrade is requested from the remote service system 28. The remote service system 28 pushes the request to the device 12, at action 236, and the device responds with an executing status, at action 238. The device 12 then extracts a web address for the file download, at action 240. In some embodiments, the web address is associated with the database 20.

At action 242, the device 12 requests the file download from the database 20, and the file is transferred to the device 12, at action 244. If the transmitted file does not match the requested file, the device 12 continues to request the file from the database 20, at action 246. In some embodiments, the device 12 requests the file up to three times before a time out operation is initiated. At action 248, the database 20 responds by transferring the correct file. When the correct file is transmitted, the device 12 applies the firmware, at action 250. If the firmware application is unsuccessful, the device 12 responds to the remote service system 28 with a "Failed" status indicating that the firmware was not installed, at action 252. If the firmware application is successful, the device 12 responds to the remote service system 28 with a "Success" status, at action 254. In some embodiments, the remote service system 28 periodically determines whether device 12 requires a software update. If a software update is required, the remote service system 28 pushes the software update to the device 12 via the service platform 18.

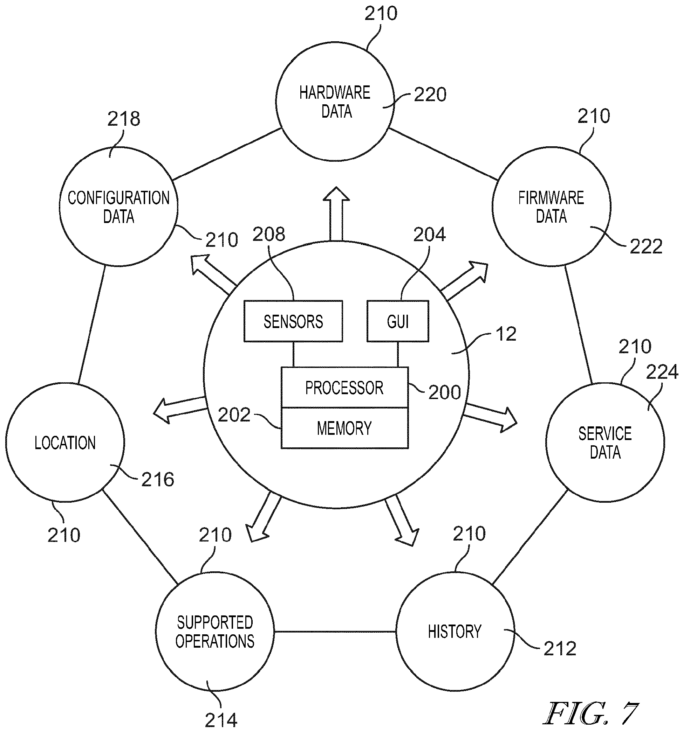

In an exemplary embodiment, a field action is initiated to update software in the field, e.g. at the facility. A list is generated of devices 12 that need to be upgraded and service orders are opened. The call center or remote service technical specialist associated with the remote service system 28 pushes software to applicable devices for each site. The customer is notified, via an on-screen pop-up on the device 12, e.g. on a GUI 204 (described below in Fig. 7), that new software is ready to apply, and the approximate time required. The customer applies new software at the device 12. The device 12 updates the remote service system 28 and the database 20 that the software has been applied. In some embodiments, the technician runs reports periodically to confirm all devices 12 included in the field action have been updated. The technician 82 closes out the service orders after confirming that the software has been applied.

Referring to Fig. 7, the device 12 includes a processor 200 electrically coupled to a memory 202. The memory 202 includes instructions that, when operated by the processor 200, control the device 12. The memory 202 also stores the data related to the device 12, e.g. the serial number, the model number, the client ID, and the external ID. A graphical user interface (GUI) 204 displays information to a user and/or technician 82. The GUI 204 includes user inputs (not shown) for operating the device 12.

The device 12 includes at least one sensor 208 for monitoring conditions of the device 12. The sensors 208 may include alarm sensors, device position sensors, e.g. bed position sensors, weigh scale sensors, battery sensors, or any other suitable sensors in a medical device. The sensors 208 transmit use data related to the device 12 to the memory 202, which stores the data. The data related to the device 12 may include a number of user alarms, changes in a bed position, user input actions, for example, user inputs, wireless connectivity events, device errors, a change in patient position and/or weight, run time on components of the device, surface state of the device, nurse call status, battery power status, and battery run time.

In some embodiments, the data related to the device 12 may include the in service date, the last date of service, the total number of days since delivery, alerts related to asynchronous errors, alerts related to bed exits, time of bed exits, alerts related to bed exits where the nurse was alerted, time of alerts related to bed exits where the nurse was alerted, alerts related to the bed malfunctioning, alerts related to the brake not being set, time that the brake was not set, voice alerts related to the brake not being set, time of voice alerts related to the brake not being set, alerts that calibration is complete, alerts regarding calibration error, an alert selected, an alert error event, alert information displayed on screen, alert input errors, alerts that an input function is unavailable, alerts related to an input success, alerts related to a nurse call confirmation, alerts that a nurse call was not connected, alerts regarding an obstacle detected on the left side of the bed, alerts regarding an obstacle detected on the right side of the bed, chair button selected, flat button selection, foot extension in button selected, foot extension out button selected, stand assist button selected, caregiver boost button selected, caregiver foot down button selected, caregiver foot up button selected, caregiver head down button selected, caregiver head up button selected, caregiver hilo down button selected, caregiver hilo up button selected, caregiver knee down button selected, caregiver knee up button selected, caregiver reverse Trendelenburg button selected, caregiver Trendelenburg button selected, patient head down button selected, patient head up button selected, patient knee down button selected, patient knee up button selected, pendant exit assist button selected, pendant foot down button selected, pendant foot up button selected, pendant head down button selected, pendant head up button selected, pendant knee down button selected, pendant knee button selected, blower level, time at blower level, CPR activation, time that backlight is dimmed, backlight activation time, pendant room light button selected, pendant room reading light button selected, caregiver lockout button selected, boost lockout activated, boost lockout time, foot lockout activated, foot lockout time, foot extend lockout activated, foot extend lockout time, head 30 degree limit lockout activated, head 30 degree limit lockout time, head lockout activated, head lockout time, hilo lockout activated, hilo lockout time, knee lockout activated, knee lockout time, foot motor activation, time that foot motor is activated, foot extension motor activated, time that foot extension motor is activated, head motor activated, time that head motor is activated, hilo foot motor activated, time that hilo foot motor is activated, hilo head motor activated, time that hilo head motor is activated, knee motor activated, time that knee motor is activated, caregiver nurse call button selected, patient nurse call button selected, pendant nurse call button selected, nurse call connected, time that nurse call is connected, foot air surface level selection, time at foot air surface level selection, head air surface level selection, time at head air surface level selection, pendant mattress firmer button selected, pendant mattress softer button selected, caregiver alarm silence button selected, exiting alarming time, exiting mode time, exiting silence expired, exiting silenced, exiting silenced time, out of bed alarming time, out of bed mode time, out of bed silenced, out of bed silenced time, position alarming time, position mode time, position silence expired, position silenced, position silenced time, foot rail down time, foot rail up time, head rail down time, head rail up time, scale calibration error for non-optimal position, scale calibration error not settled, scale calibration error timeout, scale calibration started, scale calibration success, scale new patient zeroed, scale weigh failed, scale weigh started, scale weighed, scale weight saved, scale weight too high error, scale weight too low error, scale weight unstable error, scale zero diff huge error, scale zero diff large, scale zero diff small error, scale zero failed, scale zero started, scale zeroed, air surface boost time, air surface CPR, air surface left turn time, air surface max inflate time, air surface normal, air surface right turn time, battery discharge time, bed on ac power time, bed on battery power time, bed operational time, bed shut down, bed started up, pendant TV closed-caption button selected, pendant TV channel down button selected, pendant TV channel up button selected, pendant TV mute button selected, pendant TV on off button selected, pendant TV volume down button selected, pendant TV volume up button selected, operation time of each component, cycle time of each component, number of cycles of each component, weight versus time distribution on each section of the bed, motor run times, number of motor cycles, etc.

During use of the device 12 and/or during a service call for the device 12, the device 12 is capable of transmitting data 210 that is stored in the memory 202 to the network 10. For example, the device 12 may transmit a History 212 of the device 12, wherein the history 210 includes at least any of the data listed above. Supported Operations 214 includes data related to the latest software update installed on the device 12. The Supported Operations 214 also includes data related to a software configuration of the device 12 and software that enables a remote restart of the device 12. Data related to a Location 216 of the device 12 includes a unit number, a floor number where the device is located in the facility 14, and data related to a wireless access point for the device 12. Configuration data 218 includes data related to a Wi-Fi configuration of the device 12. Hardware data 220 includes data related to a type, model, and revision of the device 12. Firmware data 222 includes data related to a software version operated on the device 12 and the components utilized in the device 12. Lastly service data 224 includes data related to error codes recorded by the device 12 and service requests.

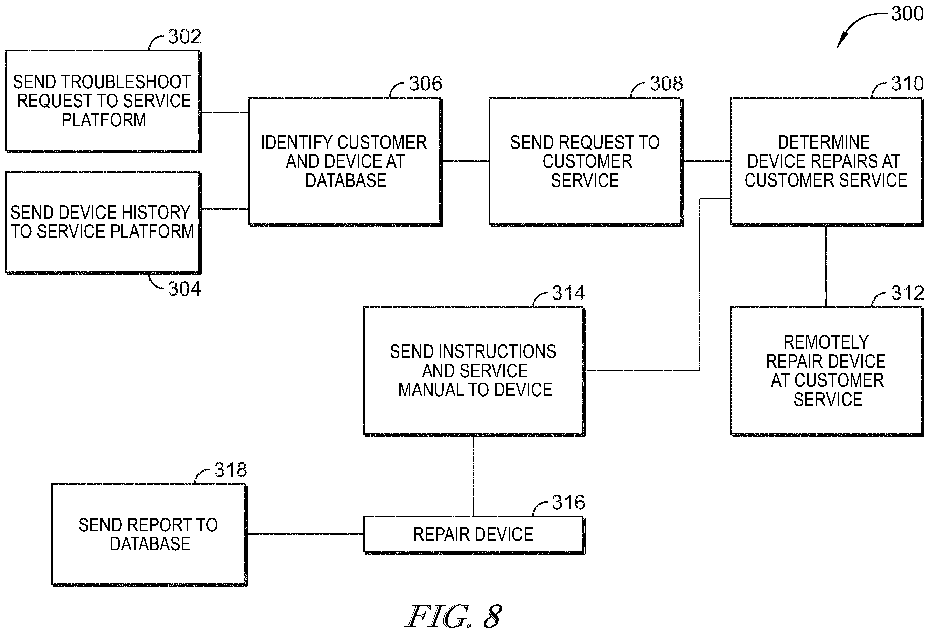

All of the data 210 is stored in the memory 202 and capable of being retrieved over the network 10. For example, the data 210 may be retrieved to troubleshoot and maintain the device 12 using the method 300 illustrated in Fig. 8. Although the method 300 is discussed with relation to the network 10 shown in Fig. 1, it will be appreciated that the method 300 is equally applicable to network 40, shown in Fig. 2. Referring to Fig. 8, at block 302, a troubleshoot request is sent from the device 12 to the remote service system 28 over the platform 18. The troubleshoot request may be a request for updated software, a request for maintenance, and/or a request for a replacement component. In some embodiments, the History 212 of the device 12 is sent from the device 12 over the platform 18 along with the troubleshoot request and an error code, at block 304. In some embodiments, at block 304, any of the data 210 related to the device 12 may be transmitted over the network 10.

At block 306, the serial number, the Device ID, and/or External ID of the device 12 is compared to data within the database 20 to identify the facility 14 that owns the device 12 and to identify the device 12 itself. The request is then transmitted to the remote service system 28 over the platform 18, at block 308. Customer service at the remote service system 28 reviews the request along with the associated data that has been transmitted to remotely to create a service order that includes repairs and/or components that will be necessary to fix the device 12, at block 310. The service order may include part numbers of the parts that are required to be replaced.

In a scenario where the device 12 may be repaired remotely, e.g. through software upgrades, the remote service system 28 transfers new software to the device 12 over the network 10, at block 312. When in person repairs are required, remote service system 28 transmits a service manual to the device 12 for review by the technician 82, at block 314. In some embodiments, only the portions of the manual pertinent to the repair are transmitted to the technician 82. In some embodiments, the manual is sent to a remote device operated by the technician 82. In other embodiments, the manual is displayed on the GUI 204 of the device 12. Additionally, at block 314, the technician 82 is transmitted instructions for repairing the device 12 and a list of parts required to make the repairs. At block 316, the technician 82 repairs the device 12. The technician 82 then sends a report over the network 10 from the device 12 that the repairs have been completed, at block 318, and the error code is cleared.

Accordingly, the device 12 contains sensors, computers, and software that can diagnose, store, and report service related information like errors, calibrations, and/or preventative maintenance needs of the device 12. The device 12 stores information in the memory 202 that can be used to identify the service procedures for the device 12, e.g. model number, serial number, date of manufacture, Device ID, External ID, etc. The device 12 can connect to the remote service system 28 to communicate the identifying information of the device 12 over the platform 18.

The remote service system 28 receives the identifying information of the device 12 and provides a service procedure to the technician 82 specific to the device 12. The remote service system 28 also provides a service order to the technician 82 that includes warranty repair information, payment information, and service contract information. In some embodiments, the technician 82 orders parts and records the work done on the product. The technician 82 accesses the remote service system 28 to access the service procedure. Alternatively, the device 12 could display the service procedure on the GUI 204.

The device 12 also includes software internal to the device 12 to log and capture events in combination with a wireless interface to the platform 18. The software captures device events that may include user alarms, change of bed position, user input actions (patient and caregiver), connectivity adds/drops, bed errors, patient position and/or weight, run time on components, e.g. actuators and/or blowers, surface state, nurse call status, AC/battery power status and run time, etc. These events are transmitted to the platform 18 and stored for either a fixed period of time or for a fixed number of data points. When a diagnostic error code occurs on the device 12, the data is bundled with that error and stored indefinitely until the error is cleared.

In some embodiments, instead of reporting just a single error code, the error code is reported in addition to the previous 1-2 hours of events or 50-100 events that preceded it. The data is used by service and engineering to retrace the activity history of the device 12 to assist in reproducing what caused the failure.

In an exemplary embodiment, a remote notification of failure for field service is received at the remote service system 28. For example, the device 12 has an electrotechnical failure and sends an error code to the remote service system 28. The remote service system 28 notifies an assigned field service technician 82 of the notification via handheld device. An error code is provided to the technician 823 with a link to a specified service procedure. The technician 82 is also provided the serial number and model number of the device 12. A location of the device 12 is then obtained from the database 20 along with a status of the device 12, e.g. whether the device 12 is occupied. At this time, the technician 82 opens a service order and orders necessary parts for the device 12. In some embodiments, the opening of the service order and ordering of parts may be automated by the remote service system 28. The technician 82 brings appropriate part, as indicated in the service procedure, to the facility 14 and repairs the device 12. Then, the technician 82 updates the service order using over the platform 18. The remote service system 28 automatically updates to indicate that the device 12 has been repaired. The service order is closed out and reviewed to determine if FDA needs to be notified of the breakdown by a quality team. Invoices may be sent by the remote service system 28.

The network 10 and the network 40 facilitate optimizing investment in assets for usage, preventive maintenance and uptime and minimize clinical disruptions in patient care areas. The network 10 and the network 40 also facilitate improving operational efficiencies by leveraging new clinical benefits and providing compliance with IT security standards. The network 10 and the network 40 provide remote fleet management, including usage and metrics. Configuration files and software updates/upgrades are configured to be deployed remotely. Additionally, automated log file collection is provided for expedited troubleshooting. The network 10 and the network 40 provide a single manufacturer/supplier service solution across all categories of devices 12. Moreover, onsite touches are minimized to ensure a first time fix of the device 12.

Any theory, mechanism of operation, proof, or finding stated herein is meant to further enhance understanding of principles of the present disclosure and is not intended to make the present disclosure in any way dependent upon such theory, mechanism of operation, illustrative embodiment, proof, or finding. It should be understood that while the use of the word preferable, preferably or preferred in the description above indicates that the feature so described can be more desirable, it nonetheless cannot be necessary.

Embodiments of the invention can be described with reference to the following numbered clauses, with preferred features laid out in the dependent clauses:
1. A system for remote diagnostics of medical devices in a healthcare network, the system comprising:
   at least one medical device having an associated serial number;
   a service platform in communication with the at least one medical device;
   a manufacturer database in communication with the service platform, the manufacturer database including data related to the at least one medical device; and
   a remote service system in communication with the service platform,
   wherein the at least one medical device sends a registration request to the manufacturer database via the service platform, wherein the registration request includes the serial number of the at least one medical device,
   upon recognizing the serial number of the at least one medical device, the manufacturer database returns unique device credentials to the at least one medical device,
   wherein the at least one medical device stores the unique device credentials, and
   wherein the remote service system identifies the at least one medical device based on the unique device credentials.
2. The system of clause 1, wherein:
   the at least one medical device sends a create device request to the remote service system via the service platform,
   the remote service system creates a local identification for the at least one medical device, and the at least one medical device stores the local identification.
3. The system of clause 1 or 2, wherein:
   the at least one medical device send an error code to the remote service system via the service platform, and
   the remote service system identifies the at least one medical device based on the unique device credentials.
4. The system of clause 3, wherein the remote service system opens a service order based on the error code and the unique device credentials.
5. The system of clause 4, wherein the remote service system identifies parts necessary to repair the at least one medical device based on the error code and the unique device credentials.
6. The system of clause 4 or 5, where the remote service system notifies a technician of the service order.
7. The system of any one of the preceding clauses, wherein:
   the at least one medical device requests a software update from the remote service system via the service platform, and
   the remote service system pushes the software update to the at least one medical device.
8. The system of clause 7, wherein the remote service system pushes the software update to a plurality of medical devices that includes the at least one medical device.
9. The system of clause 8, wherein the software update is applied to the at least one medical device by a technician at the at least one medical device.
10. The system of any preceding clause, wherein:
   the remote service system periodically determines whether the at least one at medical device requires a software update, and
   the remote service system pushes the software update to the at least one medical device via the service platform.
11. The system of any preceding clause, wherein the at least one medical device stores data related to device events.
12. The system of clause 11, wherein the device events include at least one of user alarms, change of bed position, user input actions, connectivity events, bed errors, change in patient position and weight, run time on components, surface state, nurse call status, battery power status, and battery run time.
13. The system of clause 11 or 12, wherein the data related to device events is transmitted to the remote service system via the service platform after a predetermined period of time.
14. The system of clause 11, 12, or 13, wherein the data related to device events is transmitted to the remote service system via the service platform after a predetermined number of device events.
15. The system of any one of clauses 11 to 14, wherein the data related to device events is transmitted to the remote service system via the service platform when an error code is transmitted to the remote service system.
16. The system of clause 15, wherein the data related to device events is stored until the error code is cleared.
17. The system of clause 15 or 16, wherein the data related to device events includes data related to device events that occurred within a range of 1 hour to 2 hours prior to the error code.
18. The system of clause 15, 16, or 17, wherein the data related to device events includes data related to approximately 50 -100 device events prior to the error code.
19. A method of remotely servicing medical devices in a healthcare network, the system comprising:
   sending a registration request from at least one medical device to the manufacturer database via a service platform, wherein the registration request includes the serial number of the at least one medical device,
   upon recognizing the serial number of the at least one medical device, returning unique device credentials from the manufacturer database to the at least one medical device,
   storing the unique device credentials at the at least one medical device,
   identifying the at least one medical device based on the unique device credentials at a remote service system,
   sending a create device request from the at least one medical device to the healthcare network via the service platform,
   creating a local identification for the at least one medical device at the healthcare network, and storing the local identification at the at least one medical device.
20. The method of clause 19, further comprising:
   sending an error code from the at least one medical device to the remote service system via the service platform,
   identifying the at least one medical device at the remote service system based on the unique device credentials,
   opening a service order at the remote service system based on the error code and the unique device credentials.
21. The method of clause 20, further comprising identifying at the remote service system parts necessary to repair the at least one medical device based on the error code and the unique device credentials.
22. The method of clause 19, 20, or 21, further comprising:
   requesting a software update from the remote service system via the service platform, and pushing the software update from the remote service system to the at least one medical device.
23. The method of clause 22, further comprising pushing the software update to a plurality of medical devices that includes the at least one medical device.
24. The method of any one of clauses 19 to 23, further comprising:
   periodically determining at the remote service system whether the at least one at medical device requires a software update, and
   pushing the software update to the at least one medical device via the service platform.
25. The method of any one of clauses 19 to 24, further comprising storing data related to device events at the at least one medical device.
26. The method of clause 25, further comprising transmitting the data related to device events to the remote service system via the service platform at least one of after a predetermined period of time, after a predetermined number of device events, and when an error code is transmitted to the remote service system.
27. The method of clause 25 or 26, wherein the data related to device events includes data related to device events that occurred within a range of 1 hour to 2 hours prior to the error code.
28. The method of clause 25, 26, or 27, wherein the data related to device events includes data related to approximately 50 -100 device events prior to the error code.
29. The method of any one of clauses 25 to 28, further comprising storing the data related to device events until the error code is cleared.
30. A system for remote diagnostics of medical devices in a healthcare network, the system comprising:
   at least one medical device having an associated serial number;
   a service platform in communication with the at least one medical device;
   a manufacturer database in communication with the service platform, the manufacturer database including data related to the at least one medical device; and
   a remote service system in communication with the service platform,
   wherein the at least one medical device receives unique device credentials from the manufacturer database,
   wherein the at least one medical device receives a local identification for the at least one medical device from the remote service system,
   wherein the at least one medical device sends an error code to the remote service system via the service platform, and
   wherein the remote service system identifies the at least one medical device based on the unique device credentials.
31. The system of clause 30, wherein the remote service system identifies parts necessary to repair the at least one medical device based on the error code and the unique device credentials.
32. The system of clause 30 or 31, wherein:
   the at least one medical device requests a software update from the remote service system via the service platform, and
   the remote service system pushes the software update to the at least one medical device.
33. The system of clause 32, wherein the remote service system pushes the software update to a plurality of medical devices that includes the at least one medical device.
34. The system of any one of clauses 30 to 33, wherein:
   the remote service system periodically determines whether the at least one at medical device requires a software update, and
   the remote service system pushes the software update to the at least one medical device via the service platform.
35. The system of any one of clauses 30 to 34, wherein the at least one medical device stores data related to device events.
36. The system of clause 35, wherein the device events include at least one of user alarms, change of bed position, user input actions, connectivity events, bed errors, change in patient position and weight, run time on components, surface state, nurse call status, battery power status, and battery run time.
37. The system of clause 35 or 36, wherein the data related to device events is transmitted to the remote service system via the service platform at least one of after a predetermined period of time, after a predetermined number of device events, and when an error code is transmitted to the remote service system.
38. The system of clause 35, 36, or 37, wherein the data related to device events includes data related to device events that occurred within a range of 1 hour to 2 hours prior to the error code.
39. The system of any one of clauses 35 to 38, wherein the data related to device events includes data related to approximately 50 -100 device events prior to the error code.
40. The system of any one of clauses 35 to 40, wherein the data related to device events is stored until the error code is cleared.

## Claims

1. A system for remote diagnostics of medical devices in a healthcare network, the system comprising:
at least one medical device having an associated serial number;
a service platform in communication with the at least one medical device;
a manufacturer database in communication with the service platform, the manufacturer database including data related to the at least one medical device; and
a remote service system in communication with the service platform,
wherein the at least one medical device sends a registration request to the manufacturer database via the service platform, wherein the registration request includes the serial number of the at least one medical device,
upon recognizing the serial number of the at least one medical device, the manufacturer database returns unique device credentials to the at least one medical device,
wherein the at least one medical device stores the unique device credentials, and
wherein the remote service system identifies the at least one medical device based on the unique device credentials.

2. The system of claim 1, wherein:
the at least one medical device sends a create device request to the remote service system via the service platform,
the remote service system creates a local identification for the at least one medical device, and
the at least one medical device stores the local identification.

3. The system of claim 1 or 2, wherein:
the at least one medical device sends an error code to the remote service system via the service platform,
the remote service system identifies the at least one medical device based on the unique device credentials, and
the remote service system opens a service order based on the error code and the unique device credentials.

4. The system of claim 3, wherein the remote service system identifies parts necessary to repair the at least one medical device based on the error code and the unique device credentials.

5. The system of claim 3 or 4, where the remote service system notifies a technician of the service order.

6. The system of any one of the preceding claims, wherein:
the at least one medical device requests a software update from the remote service system via the service platform,
the remote service system pushes the software update to the at least one medical device,
the remote service system pushes the software update to a plurality of medical devices that includes the at least one medical device.

7. The system of any one of the preceding claims, wherein:
the remote service system periodically determines whether the at least one at medical device requires a software update, and
the remote service system pushes the software update to the at least one medical device via the service platform.

8. The system of any one of the preceding claims, wherein the at least one medical device stores data related to device events.

9. The system of claim 8, wherein the device events include at least one of user alarms, change of bed position, user input actions, connectivity events, bed errors, change in patient position and weight, run time on components, surface state, nurse call status, battery power status, and battery run time.

10. The system of claim 8 or 9, wherein the data related to device events is transmitted to the remote service system via the service platform after a predetermined period of time.

11. The system of claim 8, 9, or 10, wherein the data related to device events is transmitted to the remote service system via the service platform after a predetermined number of device events.

12. The system of any one of claims 8 to 11, wherein the data related to device events is transmitted to the remote service system via the service platform when an error code is transmitted to the remote service system.

13. The system of claim 12, wherein the data related to device events is stored until the error code is cleared.

14. The system of claim 12 or 13, wherein the data related to device events includes:
data related to device events that occurred within a range of 1 hour to 2 hours prior to the error code; and/or
data related to approximately 50 -100 device events prior to the error code.

15. The system of any one of the preceding claims, further comprising a gateway configured to enable wireless communication of at least one legacy device with the service platform.
